# EUROPEAN PATENT APPLICATION

(11) **EP 4 316 368 A1**
(43) Date of publication of application: **07.02.2024**
(21) Application number: 23184690.8
(22) Date of filing: 11.07.2023
(51) Int. Cl.: A61B 5/12, H04R 25/00, A61B 5/00

(54) **HYBRID EAR-PROBE FIT**

(30) Priority: 05.08.2022 EP 22188953
(71) Applicant: Interacoustics A/S, 5500 Middelfart (DK)
(72) Inventor: VAN HAUEN, Sigurd, 5500 Middelfart (DK); NØRGAARD, Kren Monrad, 5500 Middelfart (DK)
(74) Representative: Demant

(57) **Abstract**

An instrument comprising an ear probe for insertion into an ear canal of a test subject is provided. The ear probe comprising an acoustic output unit comprising at least one speaker, the acoustic output unit being configured to provide at least one stimulus into the ear canal of the test subject via said speaker, an acoustic input unit comprising at least one microphone, the acoustic input unit being configured to receive a reflected part of said stimulus via said microphone and provide an electrical input signal, where the instrument further comprises a signal-processing unit connected to the acoustic output unit and to the acoustic input unit, where the signal-processing unit is configured to generate at least a first and a second stimulus and to provide said at least first stimulus and second stimulus to said acoustic output unit, and where the acoustic input unit is configured to receive a reflected part of said at least first stimulus and second stimulus. Further, an ear probe for insertion into an ear canal of a test subject is provided. Further, a method of performing an ear-probe-fit assessment is provided.

## Description

### SUMMARY

The present application relates to an instrument comprising an ear probe for insertion into an ear canal of a test subject.

The present application further relates to an ear probe for insertion into an ear canal of a test subject.

The present application further relates to a method of performing an ear-probe-fit assessment of an ear probe.

### An Instrument and an Ear Probe

Multiple audiologic measurements involve measuring the response to an acoustic stimulus using an ear probe (of an instrument) inserted in the ear canal of a test subject (e.g., a patient). The ear probe may consist of one or more miniature speakers and a microphone, housed in the ear-probe body and individually coupled to the ear canal using an ear-probe tip. The ear probe may be held in place by and acoustically sealed to the ear-canal walls using an ear tip, usually made of a soft rubber or foam, and mounted onto the ear-probe tip. The ear tip also provides a barrier between the ear-canal and the outside world and reduces the influence of environmental noise during measurements. When using rubber ear tips, a barometric seal can be achieved which allows pressurizing the ear-canal. This is needed for, e.g., tympanometry and pressurized otoacoustic-emission (OAE) measurements.

Prior to a measurement, an ear-probe-fit procedure is usually performed to determine the quality of the fit of the ear probe in the ear canal, e.g., the barometric seal, position, and/or orientation of the ear probe. This is usually done by measuring and analysing the ear-canal response to some acoustic stimulus. The parameters used to quantify the quality of ear-probe fits vary, depending on the following audiologic measurement. The ear-probe-fit procedure is used to indicate to the user (e.g., a hearing-care professional (HCP) operating the instrument) whether the acoustic conditions are appropriate for conducting the following audiologic measurement. The ear-probe-fit procedure can also be used to automatically initiate the measurement once appropriate conditions are achieved. Finally, the ear-probe-fit procedure can be configured to pass on certain *in-situ* correction values to the audiologic measurement that allow compensating for various acoustic phenomena influencing said measurement in the individual ear canal under consideration.

A common type of ear-probe-fit procedure utilizes traditional pure-tone tympanometry, e.g., using a probe-tone frequency of 226 Hz, to measure the acoustic volume of the ear canal. The measured volume is then used as an indicator of a leaky ear-probe fit in the ear canal or a blocked probe when the indicated acoustic volume is either too large or too low, respectively.

Another common type of ear-probe-fit procedure measures the ear-canal response to a transient stimulus. This method is usually used prior to an OAE measurement. Here, the transient response can be stored in the instrument memory and used for achieving similar ear-probe fits at follow-up monitoring sessions, where reproducibility of measurements is important. For transient-evoked otoacoustic emissions (TEOAE), it further allows estimating the decay time of the stimulus which is useful in order to avoid false responses in the TEOAE measurement (i.e., responses originating from a nonlinearity in the ear-probe speaker rather than the inner ear, contaminating the measurement time window).

The user is usually presented with the ear-probe-fit data on a display or an LED colour indicator. If the ear-probe-fit parameters do not fall within the range corresponding to a good fit, the user is required to alter the placement of the probe until a good ear-probe-fit is achieved.

If an audiologic measurement benefits from more than one type of ear-probe-fit procedure, the two or more types are to be performed sequentially. However, two or more ear-probe-fit procedures lead to an increased consultation time spend on each patient.

Therefore, normally, only one type of ear-probe-fit procedure is carried out, and the appropriate ear-probe-fit procedure depends on the type of audiologic measurement to follow.

In an aspect of the present application, an instrument comprising an ear probe is provided.

The ear probe may be suitable for insertion into an ear canal of a test subject (e.g., of a human or, alternatively, an animal, if required).

The ear probe may comprise an acoustic output unit.

The acoustic output unit may comprise an output transducer.

The output transducer may comprise or constitute at least one speaker (also termed a loudspeaker or a receiver).

The acoustic output unit may be configured to provide at least one (acoustic) stimulus into the ear canal of the test subject via said at least one speaker.

A stimulus may refer to an acoustic stimulus.

The acoustic output unit may be configured to provide a stimulus perceived by the test subject as an acoustic signal.

For example, the acoustic output unit may be configured to provide a first stimulus into the ear canal of the test subject via a first speaker. Further, the acoustic output unit may be configured to provide a second stimulus into the ear canal of the test subject via a second speaker.

For example, the acoustic output unit may be configured to provide a first and a second stimulus into the ear canal of the test subject via one speaker.

The acoustic output unit may comprise a wireless receiver for receiving a wireless signal comprising or representing said stimulus (sound signal).

The ear probe may comprise an acoustic input unit.

The acoustic input unit may comprise an input transducer.

The input transducer may comprise or constitute at least one microphone.

The acoustic input unit may be configured to receive a reflected part of said stimulus.

The acoustic input unit may be configured to receive a reflected part of said stimulus via said microphone.

The acoustic input unit may be configured to receive a response of said stimulus.

In other words, the response measured by the at least one microphone is the total sound pressure in the ear canal. In the approximation of the ear canal as a one-dimensional transmission line, this total sound pressure consists of an incident (or forward-propagating) and reflected (of reverse-propagating) part.

The acoustic input unit may be configured to provide an electrical input signal.

The acoustic input unit may be configured to provide an electrical input signal in response (or reaction) to receiving said reflected (or induced) part of said stimulus.

The acoustic input unit may be configured to provide an electrical input signal corresponding to and/or resulting from said reflected (or induced) part of said stimulus.

The acoustic input unit may comprise a wireless transmitter for transmitting a wireless signal comprising or representing said reflected part of said stimulus (sound signal).

The instrument may comprise a signal-processing unit.

The acoustic input unit may be configured to provide said electrical input signal to said signal-processing unit.

The signal-processing unit may be connected to the acoustic output unit and to the acoustic input unit.

The signal-processing unit may be configured to generate at least a first and a second stimulus. For example, the signal-processing unit may be configured to generate a plurality of stimuli. For example, the signal-processing unit may be configured to generate a plurality of first and second stimuli.

The signal-processing unit may be configured to provide said first stimulus and said second stimulus to said acoustic output unit.

The signal-processing unit may comprise a signal generator for generating/providing said at least one stimulus.

The signal-processing unit may comprise a signal generator for generating/providing said first and second stimulus.

The signal-processing unit (e.g., the signal generator of the signal-processing unit) may be connected to the acoustic output unit and to the speaker either via a wired connection or wirelessly.

The signal-processing unit may be connected to the acoustic input unit (and to the microphone of the acoustic input unit) either via a wired connection or wirelessly.

A wireless connection may, e.g., be based on Bluetooth technology (e.g., Bluetooth Low-Energy technology), or Ultra WideBand (UWB) technology.

The acoustic input unit may be configured to receive a reflected part of said at least first stimulus and second stimulus.

Thus, an instrument configured to carry out multiple types of ear-probe-fit procedures simultaneously is provided. Today, only one type of ear-probe fit is performed prior to an audiologic measurement to quantify the quality of the ear-probe fit. By utilizing multiple ear-probe-fit stimuli simultaneously, more data can support the quantification of the quality of the ear-probe fit and thereby increase reliability of the ear-probe fit and the subsequent audiologic measurement.

The ear probe may comprise an ear-probe body.

The ear-probe body may be suitable for (configured to) accommodating at least part of said acoustic output unit and/or of said acoustic input unit.

Accommodating may refer to (at least partly) surrounding and/or containing and/or be connected to and/or fixate at least part of said acoustic output unit and/or of said acoustic input unit.

The ear-probe body may be suitable for accommodating said microphone and said receiver.

The ear probe may comprise an ear-probe tip.

The ear-probe tip may be releasably attached/connected/engaged to/with the ear-probe body. The ear-probe tip may comprise a tip opening.

The tip opening may be configured to output said at least one stimulus.

The tip opening may be configured to receive said reflected part of said at least one stimulus. The tip opening may be arranged at a distal end of the ear-probe tip. In other words, the ear-probe tip may be arranged (located) at a distance from the ear-probe body.

A proximal end of the ear-probe tip may be releasably attached/connected/engaged to/with the ear-probe body. In other words, the proximal end may be located closer than said distal end to the ear-probe body.

The ear-probe tip may comprise a sound tube.

The sound tube may have a longitudinal axis (A).

The sound tube may be a hollow tube. In other words, the sound tube may comprise an inner opening extending at least part of the length of the sound tube. Said inner opening may be open at least towards the tip opening (distal end) of the ear-probe tip.

The sound tube may provide access (sound access) between a speaker opening and said tip opening.

Speaker opening may refer to an opening/output from the speaker of the acoustic output unit, e.g., via a sound (delivery) tube.

The sound tube may provide access (sound access) between a microphone opening and said tip opening.

Microphone opening may refer to an opening/input to the microphone of the acoustic input unit, e.g., via a sound (delivery) tube.

The sound tube may surround the speaker opening and/or the microphone opening.

The speaker opening may be arranged at a distance from said tip opening along said longitudinal axis of the sound tube.

The microphone opening may be arranged at a distance from said tip opening along said longitudinal axis of the sound tube.

The instrument may further comprise a pump.

The pump may be configured to provide a pressure higher than and/or lower than ambient pressure (e.g., approx. 1 bar), and/or configured to maintain ambient pressure in the ear canal, when the ear probe has been inserted into the ear canal of the test subject. The pump may be configured to maintain a static pressure (higher, lower, or equal to ambient pressure) for at least a pre-set time interval in the ear canal. The pump may be configured to vary the pressure in the ear canal according to a pre-set schedule.

The instrument may further comprise a pressure sensor.

The pressure sensor may be configured to measure the pressure in the ear canal of a test subject when the ear probe has been inserted into the ear canal.

The instrument may be configured to control the pressure in the ear canal of the test subject by the pump and the pressure sensor.

The instrument may further comprise a control unit. The control unit may be configured to control the pump based on the pressure measured by the pressure sensor. The control unit may be configured to control the pump according to a pre-determined schedule and based on the pressure measured in the ear canal of the test subject by the pressure sensor.

The pump and the pressure sensor may be in fluid communication with the ear canal (e.g., through the ear-probe tip of the ear probe).

For example, the pressure sensor may be connected to a tube connecting the pump to the ear canal using a T-connector and an additional piece of tube.

The instrument may be configured to operate in different modes, e.g., a normal mode and one or more specific modes depending on the audiologic measurement that follows. The mode may be selectable by a user, or automatically selectable.

The signal-processing unit may be configured to assess the quality of a fit of the ear probe. The assessment of the quality of the ear-probe fit may be based on said received reflected part of said first stimulus and second stimulus.

The assessment of the quality of the ear-probe fit may be based on said input signal received in response/reaction to providing said first stimulus and second stimulus.

The assessment of the quality of the ear-probe fit may be based on said input signal induced by said first stimulus and second stimulus.

For example, the instrument may be configured to provide an indication of the quality of the ear-probe fit. For example, the instrument may be configured to provide a visual and/or audio signal to the user in dependence of the quality of the ear-probe fit. The visual signal may, e.g., be turning on a green visual indicator in case the quality is above/below some threshold(s), and be turning on a red visual indicator in case the quality is below/above said threshold(s).

The instrument may be in communication with an auxiliary device. The auxiliary device may be configured to assess the quality of an ear-probe fit based on said received reflected part of said first stimulus and second stimulus.

The instrument may receive said assessment of quality from said auxiliary device.

For example, the instrument may be configured to provide said indication of the quality of the ear-probe fit based on the assessment of quality received from the auxiliary device.

The instrument may comprise an antenna and a transceiver circuitry for establishing a communication link to the auxiliary device. The communication link may be a wireless link. The wireless link may be based on Bluetooth technology (e.g., Bluetooth Low-Energy technology), or Ultra WideBand (UWB) technology.

For example, the auxiliary device may comprise a portable device such as a tablet, or may comprise a PC, a server device, etc.

The first stimulus may be different from the second stimulus.

The characteristics of the first stimulus may be different from the characteristics of the second stimulus.

For example, the first stimulus and the second stimulus have arbitrary time waveforms, but their time waveforms differ.

The first stimulus may comprise a first block and a second block.

The first stimulus may comprise a first part and a second part.

The second stimulus may comprise a first block and a second block.

The second stimulus may comprise a first part and a second part.

The first block/part may be located first and the second block/part may be located second on a time scale.

Each of the first block/part and the second block/part may be defined by a time interval.

The first block/part and the second block/part may be provided (played) consecutively.

The first block/part and the second block/part of the first stimulus may be aligned in time and phase.

The first block/part and the second block/part of the second stimulus may be aligned in time and phase.

The first block/part and the second block/part of the first stimulus may be aligned in time and amplitude.

The first block/part and the second block/part of the second stimulus may be aligned in time and amplitude.

The first block/part and the second block/part of the first stimulus may be aligned in time, but their phases may be inverted.

The first block/part and the second block/part of the second stimulus may be aligned in time, but their phases may be inverted.

The first block/part and the second block/part of the first stimulus may be aligned in time, but the polarity of the first block/part may be opposite to the polarity of the second block/part. For example, the phase in the first block/part is inverted compared to the phase in the second block/part, e.g., the first block/part is 180 degrees out of phase with the second block/part.

The first blocks/parts of the first and second stimuli may be aligned in time.

The second blocks/parts of the first and second stimuli may be aligned in time.

Thereby, said first blocks/parts at corresponding locations in time and said second blocks/parts at corresponding locations in time, respectively, may be compared directly.

The signal-processing unit of the instrument may generate the, at least, first stimulus and second stimulus consecutively.

The signal-processing unit of the instrument may generate a plurality of stimuli consecutively.

The signal-processing unit of the instrument may generate said stimuli (e.g., said first and second stimuli) consecutively until the ear probe is inserted correctly into the ear canal of the test subject (i.e., until an acceptable ear-probe fit is obtained).

The assessment of the quality of an ear-probe fit based on said received reflected part of said at least first stimulus and second stimulus, may comprise adding the first blocks/parts of the first and second stimuli which are aligned in time in a first block sum (Z_{A}).

The assessment of the quality of an ear-probe fit based on said received reflected part of said at least first stimulus and second stimulus, may comprise adding the second blocks/parts of the first and second stimuli which are aligned in time in a second block sum (Z_{B}).

The assessment of the quality of an ear-probe fit based on said received reflected part of said at least first stimulus and second stimulus, may comprise adding the first block sum (Z_{A}) and the second block sum (Z_{B}).

The assessment of the quality of an ear-probe fit based on said received reflected part of said at least first stimulus and second stimulus, may comprise subtracting the second block sum (Z_{B}) from the first block sum (Z_{A}).

The assessment of the quality of an ear-probe fit based on said received reflected part of said at least first stimulus and second stimulus, may comprise determining one or more audiological (audiologic) parameters based on the sum of the block sums (Z_{A} + Z_{B}).

The assessment of the quality of an ear-probe fit based on said received reflected part of said at least first stimulus and second stimulus, may comprise determining one or more audiological (audiologic) parameters based on the difference of the block sums (Z_{A} - Z_{B}).

The assessment of the quality of an ear-probe fit based on said received reflected part of said at least first stimulus and second stimulus, may comprise that the signal-processing unit compares (values of) the determined one or more audiological parameters with (values of) predetermined similar audiological parameters and provides a comparison signal.

The signal-processing unit may compare (values of) the determined one or more audiological parameters with (values of) predetermined similar audiological parameters and provides a comparison signal, on a frequency band basis.

For example, the frequency spectrum of the provided stimuli may be divided into a plurality of frequency bands, e.g., a first band, a second band, etc. Then, the signal-processing unit may compare (values of) the determined one or more audiological parameters with (values of) predetermined similar audiological parameters in a first frequency band, and similarly in the remainder frequency bands.

The signal-processing unit may generate the first stimulus and the second stimulus consecutively until the comparison signal fulfills predetermined requirements.

For example, that the comparison signal fulfills predetermined requirements may be equal to that the quality of an ear-probe fit is assessed to be sufficient for carrying out audiologic measurements with the instrument.

Fulfilling predetermined requirements may refer to that one or more comparison signals are above or below a predetermined threshold/limit/value.

For example, the predetermined requirements may be fulfilled when the measured volume of the ear canal falls within a certain range, or when the time it takes for a transient response "echo" to decay, e.g., 70 dB, is below a certain limit.

For example, the predetermined requirements may be fulfilled when a leak-free fit of the ear probe, a specific longitudinal location of the ear probe in the ear canal, or an adequately straight orientation (e.g., insertion angle) of the ear probe in the ear canal is obtained.

The at least one stimulus (e.g., the at least first stimulus and/or second stimulus) may comprise or constitute an acoustic signal (a sound signal).

The at least one stimulus may comprise or constitute a probe-tone stimulus. For example, the probe-tone stimulus may be a sinusoidal probe-tone signal. For example, the probe-tone stimulus may be a pure tone, e.g., of 226 Hz.

The at least one stimulus may comprise or constitute a transient stimulus.

The transient stimulus may comprise an abrupt onset, a short duration, and a broad spectrum. For example, said transient stimulus may be a click stimulus.

The transient stimulus may comprise instantaneous frequencies that are delayed. For example, said transient stimulus may be a chirp stimulus.

The at least one stimulus may comprise or constitute a speech signal.

In other words, the at least one stimulus may be a steady-state signal or (pseudo-random) noise, a chirp, or a click. Either type of signal may include multiple different frequencies, amplitudes, and phases, or multiple repetitions within the same block.

The ear probe may comprise a further speaker.

The ear probe may comprise two or more speakers.

Said at least one speaker may be configured to provide said first stimulus and said further speaker may be configured to provide said second stimulus.

In an aspect of the present application, an ear probe is provided.

The ear probe may be suitable for insertion into an ear canal of a test subject.

The ear probe may be suitable for use with an instrument a described above.

The ear probe may comprise an acoustic output unit comprising at least one speaker.

The acoustic output unit may be configured to provide at least one stimulus into the ear canal of the test subject via said speaker.

The ear probe may comprise an acoustic input unit comprising at least one microphone.

The acoustic input unit may be configured to receive a reflected part of said stimulus via said microphone.

The acoustic input unit may be configured to provide an electrical input signal.

The ear probe may comprise (or be connected to) a signal-processing unit connected to the acoustic output unit and to the acoustic input unit.

The ear probe may comprise a control unit connected to the acoustic output unit and to the acoustic input unit.

The control unit may be configured to receive at least a first and a second stimulus.

The control unit may be configured to provide said first stimulus and said second stimulus to said acoustic output unit.

The acoustic input unit may be configured to receive a reflected part of said at least first stimulus and second stimulus.

### A method

In an aspect, a method of performing an ear-probe-fit assessment is furthermore provided by the present application.

The method may comprise generating a first stimulus and a second stimulus by a signal-processing unit.

The method may comprise providing said first and second stimuli to an ear canal of a test subject, via at least one speaker of the ear probe.

The method may comprise receiving a stimuli response of the first and second stimuli from the ear canal of the test subject, via at least one microphone of the ear probe.

The method may comprise assessing an ear-probe fit based on the received stimuli response, by said signal-processing unit.

It is intended that some of or all the structural features of the instrument and the ear probe described above, in the `detailed description of embodiments' or in the claims, can be combined with embodiments of the method, when appropriately substituted by a corresponding process and vice versa. Embodiments of the method have the same advantages as the corresponding instrument and/or ear probe.

### Use

In an aspect, use of an instrument and an ear probe as described above, in the `detailed description' and in the claims, is moreover provided.

### A computer readable medium or data carrier

In an aspect, a tangible computer-readable medium (a data carrier) storing a computer program comprising program code means (instructions) for causing a data processing system (a computer) to perform (carry out) at least some (such as a majority or all) of the (steps of the) method described above, in the `detailed description' and in the claims, when said computer program is executed on the data processing system is furthermore provided by the present application.

### A computer program

A computer program (product) comprising instructions which, when the program is executed by a computer, cause the computer to carry out (steps of) the method described above, in the `detailed description' and in the claims is furthermore provided by the present application.

The computer may form part of the instrument or of the ear probe.

### A data-processing system

In an aspect, a data-processing system comprising a processor and program-code means for causing the processor to perform at least some (such as a majority or all) of the steps of the method described above, in the `detailed description' and in the claims, is furthermore provided by the present application.

### A system

In a further aspect, a system comprising an instrument and an auxiliary device as described above, in the `detailed description' and in the claims, is moreover provided.

The system may be adapted to establish a communication link between the instrument and the auxiliary device to provide that information/data can be exchanged or forwarded from one to the other.

### An APP

In a further aspect, a non-transitory application, termed an APP, is furthermore provided by the present application. The APP comprises executable instructions configured to be executed on an auxiliary device to implement a user interface for an instrument or system described above in the `detailed description', and in the claims.

### BRIEF DESCRIPTION OF DRAWINGS

The aspects of the disclosure may be best understood from the following detailed description taken in conjunction with the accompanying figures. The figures are schematic and simplified for clarity, and they just show details to improve the understanding of the claims, while other details are left out. Throughout, the same reference numerals are used for identical or corresponding parts. The individual features of each aspect may each be combined with any or all features of the other aspects. These and other aspects, features, and/or technical effect will be apparent from and elucidated with reference to the illustrations described hereinafter in which:
FIG. 1 shows an exemplary instrument according to the present application.
FIG. 2A and 2B show exemplary ear-probe fits with a quality being insufficient and sufficient, respectively, for carrying out audiologic measurements.
FIG. 3 shows exemplary electrical signals used for an ear-probe-fit assessment.
FIG. 4 shows exemplary recordings using a microphone of the stimuli shown in FIG. 3.
FIG. 5 shows exemplary evaluated recordings of the stimuli.

The figures are schematic and simplified for clarity, and they just show details which are essential to the understanding of the disclosure, while other details are left out. Throughout, the same reference signs are used for identical or corresponding parts.

Further scope of applicability of the present disclosure will become apparent from the detailed description given hereinafter. However, it should be understood that the detailed description and specific examples, while indicating preferred embodiments of the disclosure, are given by way of illustration only. Other embodiments may become apparent to those skilled in the art from the following detailed description.

### DETAILED DESCRIPTION

The detailed description set forth below in connection with the appended drawings is intended as a description of various configurations. The detailed description includes specific details for the purpose of providing a thorough understanding of various concepts. However, it will be apparent to those skilled in the art that these concepts may be practiced without these specific details. Several aspects of the instrument and method are described by various blocks, functional units, modules, components, circuits, steps, processes, algorithms, etc. (collectively referred to as "elements"). Depending upon a particular application, design constraints or other reasons, these elements may be implemented using electronic hardware, computer program, or any combination thereof.

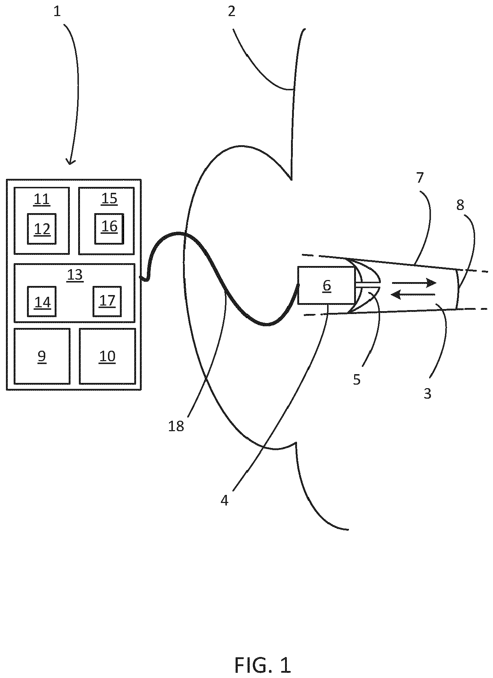

FIG. 1 shows an exemplary instrument according to the present application.

In FIG. 1, the instrument 1 is shown to be located at and in connection with the head 2 of a test subject.

The instrument 1 may be configured to test the functioning of the ear canal of the test subject, such as the middle-ear function of the test subject (e.g., in connection with tympanometry and impedance audiometry).

The instrument 1 is shown to comprise two elements, but may of course be combined to only one element, or alternatively be divided into several elements.

In FIG. 1, a first element of the instrument 1 may be arranged inside the ear canal 3 of the test subject. Alternatively, or additionally, the first element may be arranged at least partly outside the ear canal 3. The first element is shown to be an ear probe 4 suitable for insertion into the ear canal.

The ear probe 4 may comprise an ear tip 5, which may be releasably attached to an ear-probe body 6. When the ear probe 4 has been inserted correctly into the ear canal 3, the ear tip 5 may provide a barometric seal toward the ear-canal walls 7 of the ear canal 3. The instrument 1 may (via the ear probe 4) provide at least one stimulus into the ear canal of the test subject in a direction towards the eardrum 8 and receive a reflected part of said stimulus, as indicated by the two arrows located in the ear canal 3.

A second element of the instrument 1 is shown to be arranged outside the ear canal 3 of the test subject. Alternatively, or additionally, the second part (or at least some elements of the second part) could be combined with the ear probe 4.

The instrument 1 may comprise a pump 9 and a pressure sensor 10 by which the instrument 1 can control the pressure in the ear canal 3 in the space between the ear tip 5 and the eardrum 8. Therefore, the pump 9 and a pressure sensor 10 may be in fluid communication with the ear canal 3. The pump 9 and a pressure sensor 10 may provide a pressure equal to, above, or below ambient pressure in the ear canal 3.

The instrument 1 may comprise a signal-processing unit 11. The signal-processing unit 11 may comprise a signal generator 12. The signal-processing unit 11 (or the signal generator 12 of the signal-processing unit 11) may be configured to generate at least a first and a second stimulus. Said stimuli may be introduced into the ear canal 3 of the test subject via the ear probe 4.

The instrument 1 may comprise an acoustic output unit 13. The acoustic output unit 13 may be connected to the signal-processing unit 11 (and/or the signal generator 12) and may comprise at least one speaker 14 (e.g., located in the ear probe 4 even though FIG. 1 shows it in the second element). The acoustic output unit 13 may be configured to provide said first and second stimuli into the ear canal 3 of the test subject via said speaker 14.

The instrument 1 may comprise an acoustic input unit 15. The acoustic input unit 15 may be connected to the signal-processing unit 11 and may comprise a microphone 16 (e.g., located in the ear probe 4). The acoustic input unit 15 may be configured to receive a reflected part of said stimuli via said microphone 16 and provide an electrical input signal. The electrical input signal may be provided to said signal-processing unit 11.

In other words, the ear canal 3 may be stimulated with the stimuli (e.g., a first and a second stimulus) using the speaker(s) 14 and the stimuli may merge acoustically in the ear canal 3, and the acoustic response may be recorded by the microphone 16.

The signal-processing unit 11 may be configured to assess the quality of a fit of the ear probe 4 based on said received reflected part of said stimuli. In other words, the signal-processing unit 11 may be configured to assess the quality of a fit of the ear probe 4 based on the electrical input signal provided by the acoustic input unit 15.

The acoustic output unit 13 may comprise a further speaker 17 and said at least one speaker 14 may be configured to provide a first stimulus and said further speaker 17 may be configured to provide a second stimulus.

As indicated in FIG. 1, the second element may be connected to the first element. The first and second elements may be connected by a wired or a wireless connection 18.

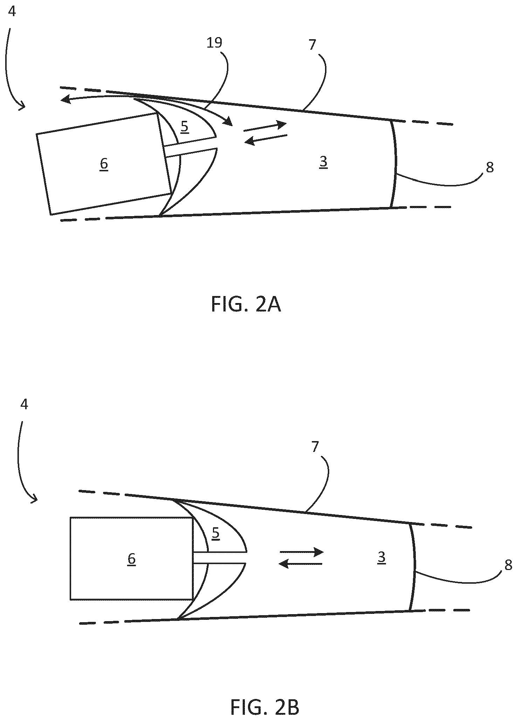

FIG. 2A and 2B show exemplary ear-probe fits with a quality being insufficient and sufficient, respectively, for carrying out audiologic measurements.

In FIG. 2A and 2B, similar features as shown in FIG. 1 are given similar reference numbers.

In FIG. 2A, an ear probe 4 is shown inserted into an ear canal 3 of a test subject. It may be a requirement that the ear probe 4 is being inserted into a specific distance from the eardrum 8. Further, it may be a requirement that the ear tip 5 provides a sufficient barometric seal toward the ear-canal walls 7 of the ear canal 3, so that, e.g., leakage of sound and/or air to and from the surroundings of the test subject does not occur.

In the specific case of FIG. 2A, the leak indicated by the double-headed arrow 19 would result in the signal-processing unit 11 assessing the quality of the ear-probe fit of the ear probe 4 to be unsatisfactory. This may, e.g., be the result when the signal-processing unit compares one or more determined audiological parameters with predetermined similar audiological parameters and provides a comparison signal, which does not fulfill predetermined requirements.

In FIG. 2B, an ear probe 4 in shown inserted into an ear canal 3 of a test subject at a location where ear tip 5 provides a sufficient barometric seal toward the ear-canal walls 7 of the ear canal 3. Thereby, no leak of sound and/or air is expected, and the signal-processing unit 11 may assess the quality of the fit of the ear probe 4 to be satisfactory. This may, e.g., be the result when the signal-processing unit compares one or more determined audiological parameters with predetermined similar audiological parameters and provides a comparison signal, which fulfills predetermined requirements.

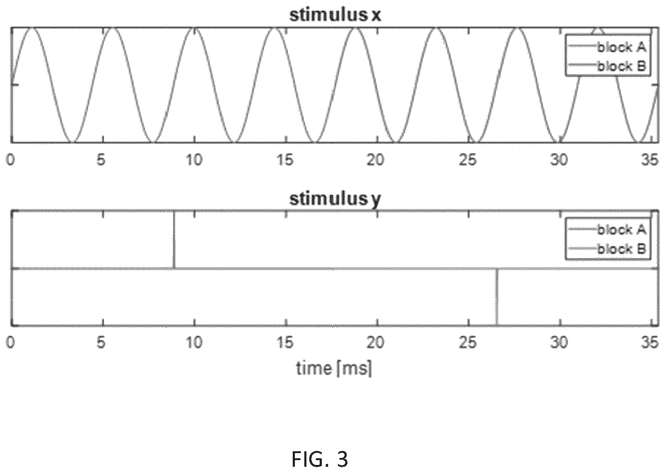

FIG. 3 shows exemplary electrical signals used for an ear-probe-fit assessment.

In FIG. 3, examples of an electrical signal of a first stimulus (stimulus x) and an electrical signal of a second stimulus (stimulus y) are shown. The stimuli are plotted as amplitude as function of time (in ms).

The first stimulus may be used for estimating the ear-canal volume using a probe tone at 226 Hz and the second stimulus may be used to record the ear-canal response to a transient stimulus.

In FIG. 3, the first stimulus (e.g., on channel 1 of a speaker) has 4 periods of a sine wave in block A (i.e., a first block/part; indicated with blue colour; from time 0 to time 18). The sine wave is repeated in block B (i.e., a second block/part; indicated with red colour; from time 18 to time 35) and is in phase with block A.

The second stimulus (e.g. a transient stimulus on channel 2 of a speaker) can be located anywhere in time in block A (blue colour; from time 0 to time 18), but must be offset by the same amount of time in block B (red colour; from time 18 to time 35) and be 180 degrees out of phase with the signal in block A.

The electrical signals are converted to soundwaves using the ear-probe speaker(s) and merge in the ear canal acoustically. The response of the soundwaves of block A and block B are recorded by the ear-probe microphone.

The duration of blocks A and B and the size of the amplitudes are only examples and may take a number of different values.

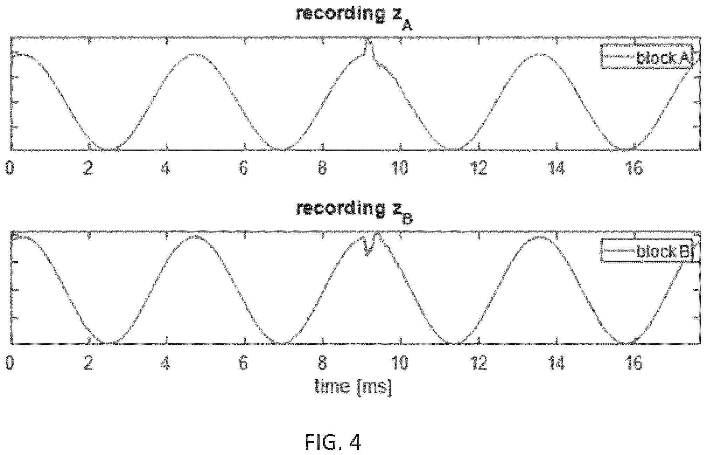

FIG. 4 shows exemplary recordings, using a microphone, of the stimuli shown in FIG. 1.

In FIG. 4, the recordings of the sine wave in block A (Z_{A}) and block B (Z_{B}) are aligned in time and in phase with each other. The transient responses, being the ripples on the peak of the 3rd sine wave, are also aligned in time, but 180 degrees out of phase with each other.

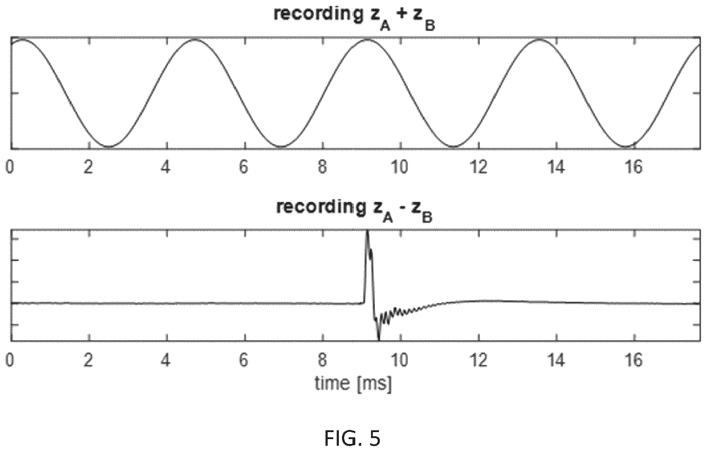

FIG. 5 shows exemplary evaluated recordings of the stimuli.

In the top graph, the recorded block A and block B responses are summed (Z_{A} + Z_{B}). The graph shows that the recorded response to the second stimulus will cancel out (will equal zero) because the responses are out of phase with each other. Thereby, only the recorded responses of the first stimulus remain, but with double amplitude.

In the bottom graph, the recorded block A and block B responses are subtracted from each other (Z_{A} - Z_{B}). After subtraction, the difference between the recorded block A and block B responses of the first stimulus will cancel out (will equal zero) because the responses are in phase. Thereby, only the recorded responses of the second stimulus remain, but with double amplitude.

Both resulting signal amplitudes can easily be scaled to the amplitude of their original respective composed signals by dividing them by 2.

From the sum of the recorded block A and block B responses, the ear-canal impedance can be calculated without the influence of the second stimulus.

Similarly, a decay analysis (or other types of analysis) of the transient response of the second stimulus can be carried out without the influence of the first stimulus.

As mentioned above, the technique is not restricted to stimuli consisting of a sine wave and a transient but could be any two (or more) types of signals and subsequent analysis.

The disclosed technique can help streamline the ear-probe fitting process and increase the number of parameters to evaluate the quality of the ear-probe fit.

It is intended that the structural features of the instrument described above, either in the detailed description and/or in the claims, may be combined with steps of the method, when appropriately substituted by a corresponding process.

As used, the singular forms "a," "an," and "the" are intended to include the plural forms as well (i.e., to have the meaning "at least one"), unless expressly stated otherwise. It will be further understood that the terms "includes," "comprises," "including," and/or "comprising," when used in this specification, specify the presence of stated features, integers, steps, operations, elements, and/or components, but do not preclude the presence or addition of one or more other features, integers, steps, operations, elements, components, and/or groups thereof. It will also be understood that, when an element is referred to as being "connected" or "coupled" to another element, it can be directly connected or coupled to the other element, but an intervening element may also be present, unless expressly stated otherwise. Furthermore, "connected" or "coupled", as used herein, may include wirelessly connected or coupled. As used herein, the term "and/or" includes any and all combinations of one or more of the associated listed items. The steps of any disclosed method are not limited to the exact order stated herein, unless expressly stated otherwise.

It should be appreciated that reference throughout this specification to "one embodiment" or "an embodiment" or "an aspect" or features included as "may" means that a particular feature, structure or characteristic described in connection with the embodiment is included in at least one embodiment of the disclosure. Furthermore, the particular features, structures or characteristics may be combined as suitable in one or more embodiments of the disclosure. The previous description is provided to enable any person skilled in the art to practice the various aspects described herein. Various modifications to these aspects will be readily apparent to those skilled in the art, and the generic principles defined herein may be applied to other aspects.

The claims are not intended to be limited to the aspects shown herein but are to be accorded the full scope consistent with the language of the claims, wherein reference to an element in the singular is not intended to mean "one and only one" unless specifically so stated, but rather "one or more." Unless specifically stated otherwise, the term "some" refers to one or more.

## Claims

1. Instrument comprising an ear probe for insertion into an ear canal of a test subject, the ear probe comprising
- an acoustic output unit comprising at least one speaker, the acoustic output unit being configured to provide at least one stimulus into the ear canal of the test subject via said at least one speaker,
- an acoustic input unit comprising at least one microphone, the acoustic input unit being configured to receive a reflected part of said at least one stimulus via said microphone and provide an electrical input signal,
- where the instrument further comprises a signal-processing unit connected to the acoustic output unit and to the acoustic input unit,
- where the signal-processing unit is configured to generate at least a first and a second stimulus and to provide said at least first stimulus and second stimulus to said acoustic output unit, and
- where the acoustic input unit is configured to receive a reflected part of said at least first stimulus and second stimulus.

2. Instrument according to claim 1, wherein the signal-processing unit is configured to assess the quality of a fit of the ear probe based on said received reflected part of said at least first stimulus and second stimulus.

3. Instrument according to any one of the preceding claims, wherein the characteristics of the first stimulus is different from the characteristics of the second stimulus.

4. Instrument according to any one of the preceding claims, wherein the first stimulus and the second stimulus each comprises a first block/part and a second block/part.

5. Instrument according to claim 4, wherein the first block and the second block of the first stimulus or of the second stimulus are aligned in time and phase.

6. Instrument according to claim 4, wherein the first block and the second block of the first stimulus or the second stimulus are aligned in time, but their phases are inverted.

7. Instrument according to any one of the preceding claims 4-6, wherein the first blocks of the first and second stimuli are aligned in time, and the second blocks of the first and second stimuli are aligned in time.

8. Instrument according to any one of the preceding claims, wherein the signal-processing unit generates the at least first stimulus and second stimulus consecutively.

9. Instrument according to any one of the preceding claims 2-8, wherein, assessing the quality of a fit of the ear probe based on said received reflected part of said at least first stimulus and second stimulus, comprises adding the first blocks of the first and second stimuli which are aligned in time in a first block sum (Z_{A}), and adding the second blocks of the first and second stimuli which are aligned in time in a second block sum (Z_{B}).

10. Instrument according to any one of the preceding claims 2-9, wherein, assessing the quality of a fit of the ear probe based on said received reflected part of said at least first stimulus and second stimulus, comprises adding the first block sum (Z_{A}) and the second block sum (Z_{B}), and subtracting the second block sum (Z_{B}) from the first block sum (Z_{A}).

11. Instrument according to any one of the preceding claims, wherein, assessing the quality of a fit of the ear probe based on said received reflected part of said at least first stimulus and second stimulus, comprises determining one or more audiological parameters based on the first block sum (Z_{A}) and/or the second block sum (Z_{B}).

12. Instrument according to claim 11, wherein the signal-processing unit compares the determined one or more audiological parameters with predetermined similar audiological parameters and provides a comparison signal.

13. Instrument according to claim 12, wherein the signal-processing unit generates the first stimulus and the second stimulus consecutively until the comparison signal fulfills predetermined requirements.

14. Instrument according to any one of the preceding claims, wherein the ear probe comprises a further speaker, and where said at least one speaker is configured to provide said first stimulus and said further speaker is configured to provide said second stimulus.

15. Ear probe (1) for insertion into an ear canal of a test subject and for use with an instrument according to any one of claims 1-14, where the ear probe comprises
- an acoustic output unit comprising at least one speaker, the acoustic output unit being configured to provide at least one stimulus into the ear canal of the test subject via said at least one speaker,
- an acoustic input unit comprising at least one microphone, the acoustic input unit being configured to receive a reflected part of said at least one stimulus via said microphone and provide an electrical input signal,
- a control unit connected to the acoustic output unit and to the acoustic input unit,
- where the control unit is configured to receive at least a first and a second stimulus and to provide said first stimulus and said second stimulus to said acoustic output unit, and
- where the acoustic input unit is configured to receive a reflected part of said at least first stimulus and second stimulus.

16. Method of performing a fit assessment of an ear probe, the method comprising:
- generating a first stimulus and a second stimulus by a signal-processing unit,
- providing said first and second stimuli to an ear canal of a test subject, via at least one speaker of the ear probe,
- receiving a response of the first and second stimuli from the ear canal of the test subject, via at least one microphone of the ear probe,
- assessing a fit of the ear probe based on the received stimuli response, by said signal-processing unit.

17. A computer program comprising instructions which, when the program is executed by a computer, cause the computer to carry out the method of claim 16.
